# EUROPÄISCHE PATENTSCHRIFT

(11) **EP 3 487 443 B1**
(45) Veröffentlichungstag und Bekanntmachung des Hinweises auf die Patenterteilung: **10.03.2021**
(21) Anmeldenummer: 17746430.2
(22) Anmeldetag: 21.07.2017
(51) Int. Cl.: A61C 9/00, A61C 8/00

(54) **VERFAHREN ZUR VERMESSUNG EINER IMPLANTAT-IMPLANTAT-SITUATION**
METHOD FOR MEASURING AN IMPLANT-IMPLANT SITUATION
PROCÉDÉ POUR MESURER LA SITUATION IMPLANT-IMPLANT

(30) Priorität: 21.07.2016 DE 102016213399
(43) Veröffentlichungstag der Anmeldung: 29.05.2019
(73) Patentinhaber: Sirona Dental Systems GmbH, 64625 Bensheim (DE)
(72) Erfinder: PFEIFFER, Joachim, 64625 Bensheim (DE); THIEL, Frank, 64372 Ober-Ramstadt (DE)
(74) Vertreter: Özer, Alpdeniz
(86) Internationale Anmeldenummer: PCT/EP2017/068541
(87) Internationale Veröffentlichungsnummer: WO 2018/015562

(56) Entgegenhaltungen:
- EP-A1- 2 177 175
- WO-A1-2009/065954
- CN-Y- 201 034 534
- DE-A1-102004 035 091
- DE-B3-102012 207 499
- GB-A- 775 876

## Beschreibung

### Technisches Gebiet

Die Erfindung betrifft ein Verfahren zur Vermessung einer Implantat-Implantat-Situation für eine Planung eines implantatgetragenen Zahnersatzteils gestützt auf mindestens zwei Implantate umfassend eine Scanschablone und die mindestens zwei gesetzten Implantate.

### Stand der Technik

Aus dem Stand der Technik sind mehrere Verfahren und Vorrichtungen zur Vermessung einer Zahnsituation bekannt.

DE 10 2012 207 499 B3 offenbart ein Verfahren zur Vermessung einer Zahnsituation umfassend mehrere Implantate, wobei unter Verwendung eines ersten Messverfahrens die gesamte Zahnsituation erfasst wird, wobei anschließend mittels eines zweiten präziseren Messverfahrens bestimmte festgelegte Objektbereiche um die Implantate vermessen werden.

Das zweite präzisere Messverfahren kann mittels eines Multikamerasystems unter Verwendung eines Fotogrammetrieverfahrens erfolgen. Zur Verbesserung der Vermessung können Messkörper verwendet werden, die Messgeometrien aufweisen, die eine Bestimmung der Lage und Orientierung der Implantate ermöglichen. Die Messgeometrie kann dabei eine bestimmte geometrische Form aufweisen. Das erste Messverfahren für die Übersichtsaufnahme kann auf einem Streifenprojektionsverfahren, auf einem konfokalen Mikroskopieverfahren, auf einem Weisslichtinterferometrieverfahren, auf einem Triangulationsverfahren mit farblichen Mustern oder auf einem dreidimensionalen Röntgenaufnahmeverfahren beruhen.

DE 10 2004 035 091 A1 offenbart ein Verfahren zur Bestimmung der Lage und Orientierung der dentalen Implantate, wobei auf das Implantat eine Messgeometrie aufgesetzt wird, die einen Rückschluss auf die Lage und Orientierung des Implantats ermöglicht.

Ein Nachteil dieser Verfahren besteht darin, dass die Genauigkeit der verwendeten Vermessungsverfahren insbesondere für die Bestimmung der Implantat-Implantat-Situation, also der relativen Lage und Ausrichtung der Implantate zueinander, nicht ausreicht.

Die Aufgabe der vorliegenden Erfindung besteht daher darin ein Vermessungssystem und ein Verfahren zur Vermessung einer Implantat-Implantat-Situation zur Verwendung bei der Planung eines implantatgetragenen Zahnersatzteils bereitzustellen, das eine genaue Vermessung und Bestimmung der Implantat-Implantat-Situation ermöglicht.

### Darstellung der Erfindung

Die Erfindung betrifft ein Verfahren zur Vermessung einer Implantat-Implantat-Situation gemäß Anspruch 1.

Die Implantat-Implantat-Situation ist als die Lage und Ausrichtung der Implantate zueinander definiert. Das implantatgetragene Zahnersatzteil kann beispielsweise eine implantatgetragene Brücke, eine implantatgetragene Brücke mit integriertem Gingivaanteil oder ein implantatgetragener Steg sein. Ein implantatgetragener Steg dient als Basiskonstruktion für steggetragene, herausnehmbare Prothesen. Das Zahnersatzteil ist dabei auf mindestens zwei Implantate gestützt, sodass zur Planung des implantatgetragenen Zahnersatzteils eine möglichst genaue Bestimmung der Lage und Ausrichtung der beiden Implantate zueinander erforderlich ist. Die Scanschablone ähnelt einer Bohrschablone, wobei die Scanschablone zur Vermessung auf die gesetzten Implantate aufgesetzt wird und anschließend zusammen mit den Implantaten vermessen wird. Diese Scanschablone kann beispielsweise unter Verwendung eines CAD/CAM-Herstellungsverfahrens aus einem formstabilen Material, wie PMMA, hergestellt sein. Die Aussparungen der Scanschablone können größer als die Implantate sein, sodass die auf die Implantate aufgesetzte Scanschablone leicht hin- und her bewegt werden kann. Die Aussparungen werden also an den Positionen der Implantate angebracht, die vor dem Setzen der Implantate beispielsweise beim Planen einer Bohrschablone festgelegt wurden. Die tatsächlichen Positionen der Implantate weichen jedoch von den geplanten Positionen ab, da beim Setzen der Implantate und durch mögliche Herstellungsfehler der Bohrschablone leichte Abweichungen von den geplanten Positionen entstehen können. Das erfindungsgemäße Verfahren dient also einer Feinvermessung, um die genaue Lage und Ausrichtung der Implantate zueinander und relativ zur Zahnsituation zu vermessen. Die ersten Markierungen sind bezüglich ihrer Form und ihrer Farbgestaltung so ausgestaltet, dass die Lage und Ausrichtung der Markierungen eindeutig bestimmt werden können. Die ersten Markierungen können beispielsweise drei punktförmige Erhöhungen sein, die in einem Dreieck angeordnet sind. Markierungen können auch andere Messgeometrien, bestehend aus geometrischen Grundformen, wie einem Quader, sein. Die Kante der Aussparung kann selbst als die erste Markierung dienen.

Denn anhand eines Verlaufs der Kante der Aussparung kann die Lage und Ausrichtung eindeutig bestimmt werden.

Ein Vorteil eines solchen Vermessungsverfahrens liegt darin, dass die Bestimmung der Lage und Orientierung der Implantate zueinander und der Implantate relativ zur Zahnsituation verbessert wird. Denn bei typischen dreidimensionalen Intraoralkameras, die auf einem optischen dreidimensionalen Vermessungsverfahren, wie einem Streifenprojektionsverfahren, beruhen, liegt die Genauigkeit der Vermessung von schwach strukturierten größeren Arealen typischerweise zwischen 50 µm und 100 µm. Diese Genauigkeit der Vermessung sinkt bei optischer Vermessung kooperativer Formen innerhalb geringer Distanz auf wenige µm. Die Lage und Ausrichtung der ersten Markierungen relativ zum nahegelegenen Implantat können also viel genauer bestimmt werden als bei einer dreidimensionalen optischen Vermessung der gesamten Zahnsituation.

Ein weiterer Vorteil eines solchen Vermessungsverfahrens liegt darin, dass die mechanische Präzision der Scanschablone höher ist als die Messgenauigkeit einer herkömmlichen optischen 3D-Kamera. Dadurch wird insbesondere die relative Lage der Aussparungen genauer vermessen. Dies ist insbesondere der Fall, wenn zwischen den gesetzten Implantaten nur Zahnfleisch liegt. Denn bei der Vermessung mittels einer herkömmlichen 3D-Kamera kann das Fehlen charakteristischer geometrischer Strukturen im Aufnahmebereich zu höheren Registrierungsfehlern und damit zu Messungenauigkeiten führen.

Dabei wird davon ausgegangen, dass die Lagebeziehung zwischen den ersten Markierungen zugehörig zu den Implantaten in der Scanschablone untereinander mit einer Genauigkeit gefertigt wurde, die oberhalb der Genauigkeit typischer 3D-Messkameras bei der Vermessung von schwach strukturierten, größeren Arealen liegt. Dies ist bei einer spanabhebenden Fertigung der Scanschablone auf einer NC-gesteuerten Bearbeitungsmaschine typischerweise gegeben. Vorteilhafterweise können die ersten Markierungen an Oberflächenbereichen um die Aussparungen angeordnet sein.

Die Oberflächenbereiche mit den Markierungen können unmittelbar am Rand der Aussparungen oder in der Nähe der Aussparungen in einem Abstand von beispielsweise maximal 5 mm zum Rand der Aussparung angeordnet sein. Vorteilhafterweise können die ersten Markierungen durch die Ränder der Aussparungen gebildet sein.

Denn die Geometrie der Ränder der Aussparungen erlaubt eine eindeutige Bestimmung der Lage und Ausrichtung der Scanschablone.

Vorteilhafterweise können die ersten Markierungen als Barcodes zur Zuordnung der einzelnen Aussparungen ausgebildet sein.

Die Barcodes können optisch erfasst werden und erlauben eine eindeutige Identifizierung der einzelnen Aussparungen. Dadurch wird also ermöglicht automatisch zu bestimmen, für welche Implantate die genaue Lage und Ausrichtung vermessen wurde. Darüber hinaus erlauben die Barcodes aufgrund ihrer Geometrie eine genaue Bestimmung der Lage und Ausrichtung der Scanschablone.

Vorteilhafterweise können im Vermessungsverfahren zusätzlich mindestens zwei an den beiden Implantaten angebrachte Scankörper verwendet werden, wobei an den Scankörpern zweite Markierungen angeordnet sind.

Die an den Implantaten angebrachten Scankörper weisen also zweite Markierungen auf, die eine genauere Bestimmung der Lage und Ausrichtung der Scankörper relativ zur Scanschablone ermöglichen. Die zweiten Markierungen können so, wie die ersten Markierungen, beliebig gestaltet sein, eine eindeutige Bestimmung der Lage und Ausrichtung zu ermöglichen.

Alternativ zu einem Scankörper kann auch ein Abutment oder eine Titanbasis verwendet werden, die mittels einer optischen 3D-Kamera vermessen werden können, um eine genaue Bestimmung der Lage und Ausrichtung relativ zur Scanschablone zu ermöglichen.

Vorteilhafterweise kann die Scanschablone unter Verwendung eines CAD/CAM-Herstellungsverfahrens mit bekannten Abmessungen hergestellt sein.

Die Scanschablone kann also wie eine Bohrschablone mittels einer herkömmlichen CAD/CAM-Vorrichtung erstellt werden. Dabei kann die Scanschablone aus einem Rohling beispielsweise mittels einer fünfachsigen CAM-Fräsmaschine hergestellt werden. Die Herstellung erfolgt also nach einem geplanten 3D-Modell mit bekannten Abmessungen. Daher sind die Abmessungen der hergestellten Scanschablone innerhalb eines Toleranzbereichs bekannt.

Vorteilhafterweise können die ersten Markierungen und/oder die zweiten Markierungen in ihrer Geometrie und/oder ihrer Farbe so gestaltet sein, dass unter Verwendung eines dreidimensionalen optischen Vermessungsverfahrens eine genaue Lage und Ausrichtung der ersten Markierungen relativ zu den zweiten Markierungen bestimmbar sind.

Dadurch wird also eine eindeutige und genaue Vermessung der Markierungen ermöglicht.

Vorteilhafterweise kann ein Durchmesser einer Aussparung größer als ein Durchmesser des entsprechenden Implantats sein.

Dadurch kann die Scanschablone auf die Implantate aufgesetzt werden, auch wenn die tatsächlichen Positionen der gesetzten Implantate von den geplanten Positionen der Implantate abweichen.

Vorteilhafterweise kann die Scanschablone aus einem formstabilen Material, wie PMMA, hergestellt sein.

Dadurch wird die Scanschablone beim Aufsetzen auf die Implantate nicht verformt, sodass die bekannten Abmessungen der Scanschablone mit den tatsächlichen Abmessungen der Scanschablone übereinstimmen.

Das Verfahren ermöglicht also unter Verwendung der Scanschablone eine genaue und eindeutige Vermessung der Lage und der Ausrichtung der Implantate relativ zueinander und relativ zur Zahnsituation. Die Zahnsituation kann dabei die benachbarten Zähne, Zahnfleischbereiche und/oder Zahnersatzteile umfassen. Das Vermessungsverfahren kann ein beliebiges Vermessungsverfahren sein, das eine eindeutige und genaue Bestimmung der Lage und Ausrichtung der ersten Markierungen relativ zu den Implantaten ermöglicht. Das Vermessungsverfahren kann beispielsweise ein Streifenprojektionsverfahren, ein konfokales Mikroskopieverfahren, ein Weisslichtinterferometrieverfahren, ein Triangulationsverfahren mit farblichen Mustern, ein taktiles Verfahren mittels eines taktilen Tastscanners oder ein dreidimensionales Röntgenaufnahmeverfahren sein.

Das Anordnen der Scanschablone relativ zu den gesetzten Implantaten kann beispielsweise durch das Verkleben der Scanschablone mit den Oberflächen der Zähne um die gesetzten Implantate und/oder durch das Verkleben der Scanschablone mit den gesetzten Implantaten und/oder durch das Verschrauben der Scanschablone mit den gesetzten Implantaten und/oder durch das Befestigen der Scanschablone an einem Kieferknochen unter Verwendung von Fixierimplantaten erfolgen.

Vorteilhafterweise können unter Verwendung des Vermessungsverfahrens auch sichtbare Oberflächen der Implantate oder darauf angebrachter Strukturen vermessen werden, wobei eine genaue Lage und Ausrichtung der Scanschablone relativ zu den Implantaten bestimmt werden.

Dadurch erfolgt die Bestimmung der relativen Lage und Ausrichtung der Scanschablone relativ zu den Implantaten ohne Verwendung von Scankörpern.

Vorteilhafterweise können an den Implantaten Scankörper angebracht werden, die zweite Markierungen aufweisen, wobei unter Verwendung des Vermessungsverfahrens die zweiten Markierungen an den Scankörpern vermessen werden, wobei eine genaue Lage und Ausrichtung der Scanschablone relativ zu den Scankörpern und damit zu den Implantaten bestimmt wird.

Dadurch wird die Bestimmung der Lage und Ausrichtung der Implantate relativ zur Scanschablone durch die Verwendung der Scankörper verbessert. Denn die Scankörper weisen die zweiten Markierungen auf, die beispielsweise bezüglich der Geometrie und der optischen Eigenschaften für eine optische Vermessung geeignet sind.

Vorteilhafterweise können anhand der bestimmten Lage und Ausrichtung der Scanschablone relativ zu den gesetzten Implantaten sowie anhand von bekannten Abmessungen der Scanschablone eine genaue Lage und Ausrichtung der Implantate relativ zueinander und/oder relativ zu einer Zahnsituation bestimmt werden.

Dadurch wird die Implantat-Implantat-Situation im Vergleich zu herkömmlichen Verfahren mit einer höheren Genauigkeit bestimmt. Denn bei einer herkömmlichen dreidimensionalen optischen Vermessung der gesamten Zahnsituation mit mehreren gesetzten Implantaten können beispielsweise Registrierungsfehler und Messfehler zu Ungenauigkeiten führen.

Vorteilhafterweise können die ermittelte Lage und Ausrichtung der Implantate relativ zueinander und/oder relativ zu einer Zahnsituation für eine Planung des implantatgetragenen Zahnersatzteils verwendet werden.

Dadurch wird also die Passgenauigkeit des herzustellenden Zahnersatzteils zu den gesetzten Implantaten verbessert.

Vorteilhafterweise kann das Vermessungsverfahren ein optisches dreidimensionalen Vermessungsverfahren oder ein taktiles Vermessungsverfahren sein.

Das optische dreidimensionale Vermessungsverfahren, wie das Streifenprojektionsverfahren, ein konfokales Mikroskopieverfahren oder ein Weisslichtinterferometrieverfahren, ist besonders vorteilhaft für eine intraorale Vermessung. Beim taktilen Vermessungsverfahren wird ein taktiler Tastscanner verwendet, der die Oberfläche des Objekts punktweise abtastet und auf diese Weise eine dreidimensionale Punktwolke des Objekts erzeugt wird. Das Vermessungsverfahren kann auch ein Fotogrammetrieverfahren sein.

Das Fotogrammetrieverfahren ist eine Messmethode und ein Auswerteverfahren der Fernerkundung, um aus Aufnahmen und genauen Messbildern eines Objektes aus unterschiedlichen Raumrichtungen seine räumliche Lage oder dreidimensionale Form zu bestimmen. Im Regelfall werden die Bilder mit einem speziellen Multikamerasystem aufgenommen. Mittels dieses Verfahrens kann aus den zweidimensionalen optischen Aufnahmen der einzelnen Kameras des Multikamerasystems eine dreidimensionale Aufnahme des aufzunehmenden Objekts berechnet werden.

Vorteilhafterweise kann die Scanschablone unter Verwendung eines CAD/CAM-Herstellungsverfahrens mit bekannten Abmessungen hergestellt werden.

Dadurch wird die Scanschablone mittels einer CAD/CAM-Vorrichtung nach einem geplanten 3D-Modell mit bekannten Abmessungen hergestellt.

Vorteilhafterweise kann die Scanschablone aus einer Bohrschablone hergestellt werden, die zum Setzen der Implantate verwendet wurde, wobei Bohrhülsen aus der Bohrschablone entfernt werden und/oder die Aussparungen angebracht werden, wobei die ersten Markierungen an den Oberflächenbereichen um die Aussparungen angebracht werden.

Dadurch wird die Scanschablone aus der bereits zum Bohren der Implantatbohrungen verwendeten Bohrschablone hergestellt.

Vorteilhafterweise kann das Anbringen der Aussparungen und der ersten Markierungen unter Verwendung des CAD/CAM-Herstellungsverfahrens durchgeführt werden.

Dadurch wird die Bohrschablone automatisch mittels einer CAD/CAM-Vorrichtung angepasst, indem die Aussparungen und die ersten Markierungen angebracht werden.

### Kurzbeschreibung der Zeichnungen

Die Erfindung wird anhand der Zeichnungen erläutert. Es zeigt, die
- Fig. 1: eine Skizze zur Verdeutlichung eines Vermessungssystems, zur Verwendung mit dem erfindungsgemäßen Vermessungsverfahren; und
- Fig. 2: eine Seitenansicht eines Ausschnitts der Scanschablone.

### Ausführungsbeispiele

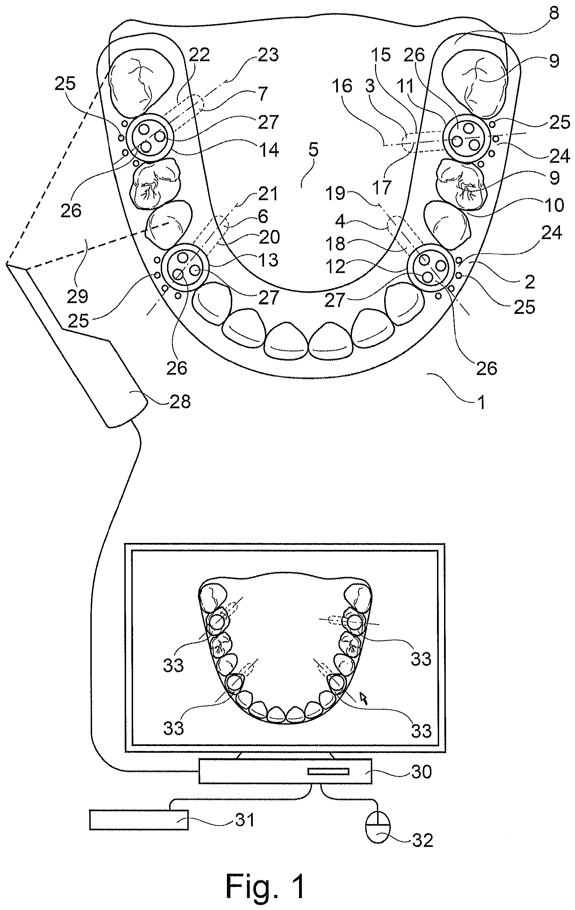

Die Fig. 1 zeigt eine Skizze zur Verdeutlichung eines Vermessungssystems 1 umfassend eine Scanschablone 2 und mindestens zwei gesetzte Implantate 3 und 4 zur Vermessung einer Implantat-Implantat-Situation 5. Im vorliegenden Fall wurden zusätzlich ein drittes Implantat 6 und ein viertes Implantat 7 gesetzt. Das vorliegende Verfahren unter Verwendung des Vermessungssystems 1 ermöglicht also eine Feinvermessung der Lage und Ausrichtung der gesetzten Implantate 4, 3, 7 und 6 relativ zueinander und relativ zur übrigen Zahnsituation 8, wobei die Zahnsituation 8 benachbarte Zähne 9 und Zahnfleischbereiche 10 umfasst. Auf die Implantate 3, 4,6 und 7 wird die Scanschablone 2 aufgesetzt, wobei an den Positionen der Implantate eine erste Aussparung 11, eine zweite Aussparung 12, eine dritte Aussparung 13 und eine vierte Aussparung 14 angeordnet sind. Das erste Implantat 3 weist also eine erste Lage 15 und eine erste Ausrichtung 16 entlang einer Symmetrieachse 17 des Implantats 4 auf. Das zweite Implantat 4 weist eine zweite Lage 18 und eine zweite Ausrichtung 19 auf. Das dritte Implantat 6 weist eine dritte Lage 20 und eine dritte Ausrichtung 21 auf. Das vierte Implantat 7 weist eine vierte Lage 22 und eine vierte Ausrichtung 23 auf. An den Oberflächenbereichen 24 um die Aussparungen 11, 12, 13 und 14 sind erste Markierungen 25 angeordnet. An den Implantaten 3, 4, 6 und 7 sind Scankörper 26 angebracht, die zweite Markierungen 27 aufweisen. Die zweiten Markierungen 27 und die ersten Markierungen 25 können bezüglich ihrer Geometrie, bezüglich ihrer Farbgestaltung und bezüglich ihrer optischen Eigenschaften so gestaltet sein, dass eine genaue und eindeutige Vermessung der Lage dieser Markierungen ermöglicht wird. Im vorliegenden Fall sind die zweiten Markierungen 27 in Form von drei halbkugelförmigen Erhöhungen ausgeführt, die in einem Dreieck angeordnet sind. Die ersten Markierungen 25 sind ebenfalls als halbkugelförmige Erhöhungen ausgeführt. Die Markierungen können auch andere geometrische Grundformen, wie eine Pyramide oder einen Quader enthalten. Die Scanschablone 2 kann beispielsweise mittels einer CAD/CAM-Vorrichtung nach einem 3D-Modell hergestellt werden. Bei der Planung der Scanschablone können beispielsweise Oberflächendaten der Zahnsituation 8 umfassend Nachbarzähne 9 sowie die aus der Implantatplanung bekannten Positionen der Implantate 3, 4, 6 und 7 verwendet werden. Der Durchmesser der Aussparungen 11, 12, 13 und 14 sind größer als der Durchmesser der gesetzten Implantate 3, 4, 6 und 7 und der aufgesetzten Scankörper 26. Die Scanschablone 2 und die an den Implantaten angebrachte Scankörper 26 werden mittels einer intraoralen 3D-Kamera 28 vermessen, die beispielsweise auf einem Streifenprojektionsverfahren beruht. Mittels der intraoralen 3D-Kamera 28 wird also ein Aufnahmebereich 29 vermessen, der durch die gestrichelten Linien dargestellt ist. Der Aufnahmebereich 29 der 3D-Kamera 28 umfasst also sowohl die ersten Markierungen 25 der Scanschablone 2 als auch die zweiten Markierungen 27 des Scankörpers 26, so dass anhand der dreidimensional optischen Aufnahme eine genaue Bestimmung der Lage und Ausrichtung der ersten Markierungen 25 relativ zum zweiten Markierungen 27 ermöglicht wird. Die Bilddaten der dreidimensionalen optischen Aufnahme der 3D-Kamera 28 werden an einen Computer 30 weitergeleitet. Am Computer 30 sind Bedienungselemente, wie eine Tastatur 31 und eine Maus 32, angeschlossen. Zur Planung eines implantatgetragenen Zahnersatzteils 33 wird also die ermittelte Lage und Ausrichtung der Implantate 3, 4, 6 und 7 relativ zueinander und relativ zur Zahnsituation 8 verwendet. Bei der Planung des Zahnersatzteils 33 wird also die äußere Geometrie in.Abstimmung zu Nachbarzähnen 9 und eine innere Geometrie in Abhängigkeit von der genauen Lage und Ausrichtung der gesetzten Implantate geplant.

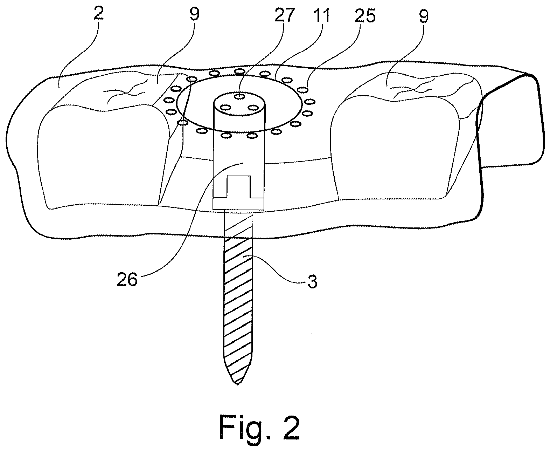

Die Fig. 2 zeigt eine Seitenansicht eines Ausschnitts der Scanschablone 2, die auf die Nachbarzähne 9 aufgesetzt ist. Auf dem ersten Implantat 3 ist der Scankörper 26 mit den zweiten Markierungen 27 angeordnet, wobei der Durchmesser der Aussparung 11 größer als der Durchmesser des Scankörpers 26 ist. Die ersten Markierungen 25 sind ringförmig in Form von halbkugelförmigen Erhöhungen am Rand der Aussparung 11 angeordnet.

### Bezugszeichen

- 1: Vermessungssystem
- 2: Scanschablone
- 3: erstes Implantat
- 4: zweites Implantat
- 5: Implantat-Implantat-Situation
- 6: drittes Implantat
- 7: viertes Implantat
- 8: Zahnsituation
- 9: benachbarte Zähne
- 10: Zahnfleischbereiche
- 11: erste Aussparung
- 12: zweite Aussparung
- 13: dritte Aussparung
- 14: vierte Aussparung
- 15: erste Lage
- 16: erste Ausrichtung
- 17: Symmetrieachse
- 18: zweite Lage
- 19: zweite Ausrichtung
- 20: dritte Lage
- 21: dritte Ausrichtung
- 22: vierte Lage
- 23: vierte Ausrichtung
- 24: Oberflächenbereiche
- 25: erste Markierungen
- 26: Scankörper
- 27: zweite Markierungen
- 28: intraorale 3D-Kamera
- 29: Aufnahmebereich
- 30: Computer
- 31: Tastatur
- 32: Maus
- 33: Zahnersatzteil

## Patentansprüche

1. Verfahren zur Vermessung einer Implantat-Implantat-Situation (5), also der relativen Lage und Ausrichtung der Implantate zueinander, zur Planung eines implantatgetragenen Zahnersatzteils (33), **dadurch gekennzeichnet, dass** hierzu eine Scanschablone (2) verwendet wird, wobei die Scanschablone (2) Aussparungen (11, 12, 13, 14) für die einzelnen Implantate (3, 4, 6, 7) und erste Markierungen (25) aufweist, wobei die Scanschablone (2) relativ zu den gesetzten Implantaten (3, 4, 6, 7) fest angeordnet wird und unter Verwendung eines Vermessungsverfahrens die ersten Markierungen (25) an der Scanschablone (2) vermessen werden.

2. Verfahren nach Anspruch 1, **dadurch gekennzeichnet, dass** unter Verwendung des Vermessungsverfahrens auch sichtbare Oberflächen der Implantate (3, 4, 6, 7) oder darauf angebrachter Strukturen vermessen werden, wobei eine genaue Lage und Ausrichtung der Scanschablone (2) relativ zu den Implantaten (3, 4, 6, 7) bestimmt wird.

3. Verfahren nach Anspruch 1, **dadurch gekennzeichnet, dass** an den Implantaten (3, 4, 6, 7) Scankörper (26) angebracht werden, die zweite Markierungen (27) aufweisen, wobei unter Verwendung des Vermessungsverfahrens die zweiten Markierungen (27) an den Scankörpern (26) vermessen werden, wobei eine genaue Lage und Ausrichtung der Scanschablone (2) relativ zu den Scankörpern (26) und damit zu den Implantaten (3, 4, 6, 7) bestimmt wird.

4. Verfahren nach einem der Ansprüche 1-3, **dadurch gekennzeichnet, dass** anhand der bestimmten Lage und Ausrichtung der Scanschablone (2) relativ zu den gesetzten Implantaten (3, 4, 6, 7) sowie anhand von bekannten Abmessungen der Scanschablone (2) eine genaue Lage und Ausrichtung der Implantate (3, 4, 6, 7) relativ zueinander und/oder relativ zu einer Zahnsituation (8) bestimmt wird.

5. Verfahren nach Anspruch 4, **dadurch gekennzeichnet, dass** die ermittelte Lage und Ausrichtung der Implantate (3, 4, 6, 7) relativ zueinander und/oder relativ zu einer Zahnsituation (8) für eine Planung des implantatgetragenen Zahnersatzteils (33) verwendet werden.

6. Verfahren nach einem der Ansprüche 1-5, **dadurch gekennzeichnet, dass** das Vermessungsverfahren ein optisches dreidimensionales Vermessungsverfahren oder ein taktiles Vermessungsverfahren ist.

7. Verfahren nach einem der Ansprüche 1-6, **dadurch gekennzeichnet, dass** die Scanschablone (2) unter Verwendung eines CAD/CAM-Herstellungsverfahrens mit bekannten Abmessungen hergestellt wird.

8. Verfahren nach einem der Ansprüche 1-6, **dadurch gekennzeichnet, dass** die Scanschablone (2) aus einer Bohrschablone hergestellt wird, die zum Setzen der Implantate verwendet wurde, wobei Bohrhülsen aus der Bohrschablone entfernt werden und/oder die Aussparungen angebracht werden, wobei die ersten Markierungen (25) an den Oberflächenbereichen (24) um die Aussparungen (11, 12, 13, 14) angebracht werden oder die Ränder der Aussparungen als Markierungen verwendet werden.

9. Verfahren nach Anspruch 8, **dadurch gekennzeichnet, dass** das Anbringen der Aussparungen (11, 12, 13, 14) und der ersten Markierungen (25} unter Verwendung des CAD/CAM-Herstellungsverfahrens durchgeführt wird.

10. Verfahren nach Anspruch 8, **dadurch gekennzeichnet, dass** ein Durchmesser einer Aussparung (11, 12, 13, 14) größer als ein Durchmesser des entsprechenden Implantats (3, 4, 6, 7) ist, so dass die auf die Implantate (3, 4, 6, 7) aufgesetzte Scanschablone (2) hin- und her bewegbar ist.

11. Verfahren nach Anspruch 10, **dadurch gekennzeichnet, dass** die ersten Markierungen (25) als Barcodes zur Zuordnung der einzelnen Aussparungen (11, 12, 13 ,14) ausgebildet sind.

## Claims

1. A method for measuring an implant-implant situation (5), that is to say the relative position and orientation of the implants with respect to one another, for planning an implant-borne tooth replacement part (33), **characterised in that** a scanning template (2) is used for this purpose, wherein the scanning template (2) has recesses (11, 12, 13, 14) for the individual implants (3, 4, 6, 7) and first markings (25), wherein the scanning template (2) is firmly arranged relative to the placed implants (3, 4, 6, 7) and the first markings (25) on the scanning template (2) are measured using a measurement method.

2. The method according to claim 1, **characterised in that** visible surfaces of the implants (3, 4, 6, 7) or structures applied thereto are also measured using the measurement method, wherein an exact position and orientation of the scanning template (2) relative to the implants (3, 4, 6, 7) is determined.

3. The method according to claim 1, **characterised in that** scan bodies (26) are applied to the implants (3, 4, 6, 7), said scan bodies having second markings (27), wherein the second markings (27) on the scan bodies are measured using the measurement method (26), wherein an exact position and orientation of the scanning template (2) relative to the scan bodies (26) and thus to the implants (3, 4, 6, 7) is determined.

4. The method according to any one of claims 1-3, **characterised in that** an exact position and orientation of the implants (3, 4, 6, 7) relative to one another and/or relative to a tooth situation (8) is determined on the basis of the determined position and orientation of the scanning template (2) relative to the placed implants (3, 4, 6, 7) and on the basis of known dimensions of the scanning template (2).

5. The method according to claim 4, **characterised in that** the ascertained position and orientation of the implants (3, 4, 6, 7) relative to one another and/or relative to a tooth situation (8) are used for planning the implant-borne tooth replacement part (33).

6. The method according to any one of claims 1-5, **characterised in that** the measurement method is an optical three-dimensional measurement method or a tactile measurement method.

7. The method according to any one of claims 1-6, **characterised in that** the scanning template (2) is produced with known dimensions using a CAD/CAM manufacturing process.

8. The method according to any one of claims 1-6, **characterised in that** the scanning template (2) is produced from a drilling template which has been used to place the implants, wherein drilling sleeves are removed from the drilling template and/or the recesses are applied, wherein the first markings (25) are applied to the surface regions (24) around the recesses (11, 12, 13, 14) or the edges of the recesses are used as markings.

9. The method according to claim 8, **characterised in that** the recesses (11, 12, 13, 14) and the first markings (25) are applied using the CAD/CAM manufacturing process.

10. The method according to claim 8, **characterised in that** a diameter of a recess (11, 12, 13, 14) is greater than a diameter of the corresponding implant (3, 4, 6, 7) so that the scanning template (2) placed on the implants (3, 4, 6, 7) can be moved back and forth.

11. The method according to claim 10, **characterised in that** the first markings (25) are designed as barcodes for assigning the individual recesses (11, 12, 13, 14).

## Revendications

1. Procédé pour mesurer la situation implant-implant (5), c'est-à-dire la position relative et l'alignement des implants les uns par rapport aux autres, pour planifier une pièce de prothèse dentaire implanto-portée (33), **caractérisé en ce qu'**un gabarit de numérisation (2) est utilisé à cet effet, le gabarit de numérisation (2) présentant des évidements (11, 12, 13, 14) pour les implants individuels (3, 4, 6, 7) et des premiers marquages (25), le gabarit de numérisation (2) par rapport aux implants (3, 4, 6, 7) étant disposé à demeure et les premiers marquages (25) sur le gabarit de numérisation (2) étant mesurés en utilisant un procédé de mesure.

2. Procédé selon la revendication 1, **caractérisé en ce que**, en utilisant le procédé de mesure, les surfaces visibles des implants (3, 4, 6, 7) ou des structures qui leur sont attachées sont également mesurées, une position et un alignement exacts du gabarit de numérisation (2) par rapport aux implants (3, 4, 6, 7) étant déterminés.

3. Procédé selon la revendication 1, **caractérisé en ce que** des corps de numérisation (26), qui présentent des seconds marquages (27), sont fixés aux implants (3, 4, 6, 7), les seconds marquages (27) sur les corps de numérisation (26) étant mesurés en utilisant le procédé de mesure, une position et un alignement exacts du gabarit de numérisation (2) par rapport aux corps de numérisation (26) et ainsi aux implants (3, 4, 6, 7) étant déterminés.

4. Procédé selon l'une quelconque des revendications 1-3, **caractérisé en ce que** sur la base de la position et de l'orientation déterminées du gabarit de numérisation (2) par rapport aux implants (3, 4, 6, 7) placés et sur la base des dimensions connues du gabarit de numérisation (2), la position et l'alignement des implants (3, 4, 6, 7) les uns par rapport aux autres et/ou par rapport à une situation dentaire (8) étant déterminés.

5. Procédé selon la revendication 4, **caractérisé en ce que** la position et l'orientation déterminées des implants (3, 4, 6, 7) les uns par rapport aux autres et/ou par rapport à une situation dentaire (8) sont utilisées pour planifier la pièce de prothèse dentaire implanto-portée (33).

6. Procédé selon l'une quelconque des revendications 1-5, **caractérisé en ce que** le procédé de mesure est un procédé de mesure optique tridimensionnel ou un procédé de mesure tactile.

7. Procédé selon l'une quelconque des revendications 1-6, **caractérisé en ce que** le gabarit de numérisation (2) est fabriqué avec des dimensions connues en utilisant un procédé de fabrication CAD/CAM.

8. Procédé selon l'une quelconque des revendications 1-6, **caractérisé en ce que** le gabarit de numérisation (2) est fabriqué à partir d'un gabarit de fraisage qui a été utilisé pour poser les implants, les manchons de fraisage étant retirés du gabarit de fraisage et/ou les évidements étant réalisés, les premiers marquages (25) étant fixés sur les zones superficielles (24) autour des évidements (11, 12, 13, 14) ou les bords des évidements étant utilisés comme marquages.

9. Procédé selon la revendication 8, **caractérisé en ce que** les évidements (11, 12, 13, 14) et les premiers marquages (25) sont réalisés en utilisant le procédé de fabrication CAD/CAM.

10. Procédé selon la revendication 8, **caractérisé en ce que** le diamètre d'un évidement (11, 12, 13, 14) est supérieur au diamètre de l'implant (3, 4, 6, 7) correspondant, de sorte que le gabarit de numérisation (2) disposé sur les implants (3, 4, 6, 7) peut être déplacé dans un sens et dans l'autre.

11. Procédé selon la revendication 10, **caractérisé en ce que** les premiers marquages (25) sont réalisés sous forme de codes-barres pour attribuer les évidements individuels (11, 12, 13, 14).
